(19) Europäisches Patentamt / European Patent Office / Office européen des brevets

(11) **EP 4 700 389 A1**

(12) **EUROPEAN PATENT APPLICATION**

(43) Date of publication:
**25.02.2026 Bulletin 2026/09**

(21) Application number: **24195646.5**

(22) Date of filing: **21.08.2024**

(51) International Patent Classification (IPC):
**G01N 33/53** $^{(2006.01)}$   **C07K 7/06** $^{(2006.01)}$
**C07K 7/08** $^{(2006.01)}$

(52) Cooperative Patent Classification (CPC):
**G01N 33/5308;** C07K 7/06; C07K 7/08

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicants:
• **Université de Liège**
  **4000 Liège (BE)**
• **Buildwise**
  **1932 Zaventem (BE)**

(72) Inventors:
• **Lassaux, Patricia**
  **4000 Liège (BE)**
• **Nicaise, Dominique**
  **1932 Zaventem (BE)**

(74) Representative: **Gevers Patents**
  **De Kleetlaan 7A**
  **1831 Diegem (BE)**

Remarks:
The complete document including Reference Table(s) and the Sequence Listing(s) can be downloaded from the EPO website

(54) **ASBESTOS DETECTION**

(57) The present invention relates to a method for the detection of asbestos in a sample.

EP 4 700 389 A1

**Description**

**Technological Background of the Invention**

The present invention relates to an improved biological method for the detection of the presence of asbestos in a sample, more specifically a method making use of bioengineered peptides that specifically bind to selected asbestos fibers ("asbestos-binding peptides") in a sample. In a further aspect, the present invention relates to asbestos-binding peptides as well as to a kit for the detection of the presence of asbestos in a sample.

Asbestos is a general term covering different types of naturally existing fibrous silicatebased minerals. Six different types of asbestos classified in two different mineral groups have been acknowledged so far: the chrysotile type, part of the serpentine mineral group, and the amosite type, the crocidolite type, the tremolite type, the actinolite type and the anthophyllite type, all part of the amphibole mineral group. These different types differ by composition and shape. The chrysotile form accounts for 94% of the worldwide asbestos market.

Asbestos is known for its mechanical strength, chemical and thermal stability, more specifically resistance to fire. Thanks to these characteristics, it has been used in many applications ranging from car brake pads, fire blankets, filters, construction materials, concrete reinforcement, window sills, building panels, construction bricks and blocks, ceiling panels, plaster and plaster boards, roof tiles, roof and wall covers, water and sewage pipes to insulating material. These materials may comprise from about 2 to 20 % wt. of asbestos fibers. Nevertheless, given its hazardous nature, it has been prohibited in many countries. In fact, asbestos fibers are extremely thin (often less than 0.1 $\mu$m) and known as a carcinogenic agent, more specifically for their tendency to cause pleural mesothelioma and lung cancer, particularly because of their capacity to easily become air borne and inhaled.

Even in countries where it has been prohibited, many constructions still comprise asbestos-containing materials which can present a risk for residents and workers, more specifically in case of refurbishment of older buildings. In situations where the presence of asbestos is suspected, the construction site is put on standstill until test results from relevant analyses become available and decision is taken on how to proceed further in order to reduce the risk of exposure, particularly worker exposure, to carcinogenic airborne asbestos fibers.

There is thus a need for effective and reliable detection methods that are capable of generating results rapidly to confirm the presence or absence of asbestos.

**Prior Art**

Different methods for asbestos detection are known. Laboratory methods include optical and electronic microscopy and X-ray diffraction. As well, in situ detection method, using anear-infrared (NIR) detection device (PhazIR™, Polychromix, USA) has been developed for in situ determination of the presence of asbestos. This method nevertheless still requires specifically trained personnel for the performance of the relevant tests. Also, the quantitative determination of asbestos requires additional resources.

There is thus an ongoing interest in easy-to-use asbestos detection methods and kits, more specifically such methods and kits that allow for in-situ use with fast results.

With this aim in mind, EP-1953549 proposes contacting an asbestos-binding protein in a solution containing at least 0.1 M sodium chloride with asbestos in a sample and to detect the presence of this protein fused to a reporter protein. In the examples, data is provided for DksA protein from *Escherichia coli* as asbestos-binding protein. The detection method described is a colorimetric detection method making use of a DksA fused to alkaline phosphatase. In an alternative method, the DksA is fused to Green Fluorescent Protein (GFP) allowing the detection of chrysotile type asbestos by fluorescence microscopy. Ishida et al., Selective detection of airborne asbestos fibers using protein-based fluorescent probes. Environ Sci Technol. 2010 Jan 15; 44(2):755-9 then went on to use a bacterial protein called GatZ in a fluorescence based method to selectively determine the presence of amosite and crocidolite.

T Ishida et al, Molecular Engineering of a Fluorescent Bioprobe for Sensitive and Selective Detection of Amphibole Asbestos, Plos One. September 2013, vol 8, Issue 9, e76231, found that GatZ and H-NS protein were not sufficiently specific for amphibole type asbestos as they also bind to wollastonite, an asbestos substitute. The authors speculate that GatZ protein fragments are unlikely to be suitable and propose to look for H-NS protein fragments that specifically bind to amphibole asbestos fibers represented by amosite type asbestos fibers. After evaluating several fragments, the authors found that H-NS$_{60-90}$ specifically binds to amosite type asbestos without binding to wollastonite. The fragments, however, showed rather weak affinity to amosite and the authors had to engineer streptavidin-based protein fragment tetramer in order to improve the protein fragment affinity to amosite asbestos. The authors went on to further engineer the peptides but stick to the idea of engineering streptavidin based tetramers showing multiple binding sequences. Also, the authors conclude that the presence of lysine side chains is the most important determinant of the peptide interaction with asbestos.

The proposed detection methods appear to make use of complex engineered molecules to come to a selectivity towards amphibole asbestos.

Efforts have also been directed at identifying small peptides that bind with high affinity to inorganic materials. These specific peptides are called "genetically engineered peptide or polypeptide for inorganics". They are defined as a sequence of amino acids that specifically and selectively binds to an inorganic surface. Whereas the molecular mechanisms of peptide recognition and binding to solid materials are not well understood, some studies showed that the binding is largely governed by electrostatic interactions.

An advantage of inorganic-binding peptides is their high specificity that depends on (i) the chemical composition, (ii) the structuration (powder or flat surface) and the (iii) crystallographic form of the inorganic target substrate. This specificity gives them interesting properties and several applications have already been highlighted (C Tamerler et al, Molecular biomimetics: utilizing nature's molecular ways in practical engineering, Acta biomaterialia, 2007, 3(3) 289-99). Inorganic-binding peptides display useful characteristics and could be used as molecular erectors to direct the assembly of hybrid materials with control of composition and topography. Genetic fusion of these inorganic-binding peptides to functional proteins enables them to be used as linkers for many bio-nanotechnological or microelectronic applications. Inorganic-binding peptides may also be used for self-oriented immobilization of enzymes with conservation of their enzymatic activity. A fivefold repeat of goldbinding-peptide has been fused to the alkaline phosphatase enzyme. The resulting bifunctional enzyme is then self-immobilized, homogenously covering the gold substrate.

Document EP-3770599 discloses a method for the detection of asbestos in a sample, comprising contacting a sample with one or more asbestos-binding peptides having a specific amino acid sequence and/or asbestos-binding peptides having a specific motif selected from S/T X S/T or S/T XX S/T. However, while it was quite selective towards pure asbestos fibers and pure non-asbestos fibers, the method of EP-3770599 is not satisfying when using natural materials containing raw materials or a mixture of materials.

**Aims of the Invention**

The purpose of the present invention is to provide an improved method using an asbestos-binding peptide for the detection of asbestos in a sample, preferably in the form of a dispersion or suspension in a liquid medium, such as a sample of construction material or of biological material or of ground or an air sample.

Another aim of the invention is to provide a simple detection method that allows easy *in situ* determination of the presence or absence of asbestos, particularly on the field.

The method should demonstrate an increased specificity for asbestos type or types commonly used in construction materials, which is essentially the chrysotile type. Preferably, it should also detect amphibole types of asbestos. The method needs to increase the selectivity towards other fibers, such as glass fibers or stone or glass wool fibers.

Advantageously, the material according to the present invention enables detection of asbestos fibers in raw samples and/ or in samples containing a mixture of fibres.

In addition, the invention seeks to provide a novel asbestos-binding peptide specifically and selectively binding to asbestos substrates.

A further purpose of the present invention is to provide a kit including said asbestos-binding peptide according to the invention for the detection of asbestos fibers in a sample, the kit being easy to use and suitable for on-site use. Further, the kit should give a result in a short period of time.

**Description of the Invention**

Accordingly, the present invention provides a method for the detection of asbestos in a sample, comprising:

(a) contacting a sample with at least one asbestos-binding peptide comprising the sequence T G A A E V A M M S K R [SEQ ID NO:1], and
(b) detecting the presence of said at least one asbestos-binding peptide bound to asbestos fibers.

It has been found that the method as described above allows for an increased/ an improved specific binding of the relevant peptides to asbestos fibers.

In the context of this description, the terms "asbestos-binding peptide" define a peptide that binds to asbestos fibers with a sufficient affinity to be measurable with known techniques; and with a selectivity such that binding to undesirable species, such as glass fibers, stone or glass wool, is null or negligible, that is that non-specific binding to undesirable species remains below a level that would be detectable by the detection method used, more particularly below a level detectable by the human eye in fluorescent or colorimetric detection methods.

Preferably, according to the invention, said method for the detection of asbestos comprises a step to separate said at least one bound asbestos-binding peptide from unbound asbestos-binding peptides such as a washing step performed with for example distilled water or a buffer solution. By including an appropriate step to separate bound peptides from unbound peptides, for instance a step aiming at removing unbound peptides, such as a washing step, the peptides bound to asbestos fibers may be retained and may be detected by appropriate detection methods, such as fluorescence techniques, colorimetric techniques and other methods known per se and within the ambit of the knowledge of the skilled person. Advantageously, according to the invention, detecting the presence of said at least one asbestos-binding peptide bound to asbestos fibers comprises fluorescence techniques.

Preferably, according to the invention, detecting the presence of said at least one asbestos-binding peptide bound to asbestos fibers comprises colorimetric techniques.

Advantageously, according to the invention, one or more asbestos-binding peptide is coupled, possibly in tandem repeats, to a reporter sequence (amino acid sequence), such as a reporter sequence selected from fluorescent protein, luciferase, alkaline phosphatase, beta-galactosidase, beta-lactamase, diaphorase and peroxidase, maltose-binding protein, laccase, lipase, and glucoside hydrolase, or coupled, possibly in tandem repeats, to a reporter molecule selected from fluorescent dyes or pigments, such as fluorescein, Cy3, DyLight488, Alexa488, ATTO488, CF488A, DyLight550, CF555, Cy2, rhodamine green, rhodamine, Cy5, Alexa488, Alexa546, Alexa555, Alexa568, Alexa594, CF568, CF594 and DY547.

In other words, the asbestos-binding peptide according to the invention may advantageously be coupled to a reporter molecule. Known reporter molecules may advantageously be selected from fluorescent dyes or pigments, such as fluorescein, Cy3, DyLight488, ATTO488, CF488A, DyLight550, CF555, Cy2, rhodamine green, rhodamine, Cy5, Alexa (Alexa488, Alexa546, Alexa555, Alexa568, Alexa594), CF568, CF594 and DY547. Preferably, said asbestos-binding peptide according to the invention coupled to a reporter molecule retains its ability in contacting a sample. In an alternative, said asbestos-binding peptide according to the invention may advantageously be coupled, possibly in tandem repeats, to a reporter sequence (amino acid sequence) known in the art. Such reporter sequence may advantageously be selected from fluorescent protein, luciferase, alkaline phosphatase, beta-galactosidase, beta-lactamase, diaphorase, peroxidase, maltose-binding protein, laccase, lipase, and glucoside hydrolase. Preferably, according to the invention, the asbestos-binding peptide coupled, possibly in tandem repeats, to a reporter sequence (amino acid sequence) known in the art, retains its ability in contacting a sample.

According to the present invention, the term "coupled" means "combined". In this sense the terms "asbestos-binding peptide coupled to a reporter sequence (amino acid sequence)" mean that the asbestos-binding peptide is combined to a means allowing the detection of said asbestos-binding peptide. For example, according to the invention, the means to detect said at least one asbestos binding-peptide can be coupled, fused or attached to said at least one asbestos-binding peptide.

Advantageously, according to the invention, at least one asbestos-binding peptide is coupled to an enzyme such as a reporter sequence enzyme, the method comprising an additional step wherein one or more reporter substrate contacts said at least one asbestos-binding peptide coupled to an enzyme. More preferably, the reporter substrate is a chromogenic substrate or a fluorescent probe. Depending on the reporter sequence, a reporter substrate is used to generate a signal. For example, the reporter sequence may be an enzyme and the reporter substrate may be a substrate for the enzyme. The asbestos-binding peptide according to the invention may be obtained by techniques known per se. It may for instance be synthesized chemically or produced in organisms, such as bacteria or yeast. The relevant asbestos-binding peptide may be isolated by phage display from appropriate libraries as will be described in more details below. Other techniques known by the skilled person may also be used.

As is generally known in the art, the asbestos-binding peptide may comprise an amidation at the C-terminal end of the amino acid sequence.

Advantageously, said at least one asbestos-binding peptide according to the invention is placed in solution, preferably water or a polar organic solvent, most preferably water.

Preferably, according to the invention, the sample is a sample of construction material, an air sample, a ground sample or a sample of biological material. The sample may be a sample of car brake pads, fire blankets, filters, construction materials, concrete reinforcement, window sills, building panels, construction bricks and blocks, ceiling panels, plaster and plaster boards, roof tiles, roof and wall covers, soil, mine, dust, burnt products, water and sewage pipes or insulating material or another construction material. Preferably, the sample comprises material collected from the surface of the relevant material to be tested or alternatively from bulk construction material. In the alternative, the sample may be an air sample, that is a sample of air taken at a location where air borne asbestos fibers are suspected, for instance close to a site under construction or renovation. According to a different aspect, the sample may be biological material, such as a biopsy or a post mortem body sample. For the purposes of this patent application, the sample is preferably in the form of a dispersion or suspension in a liquid medium.

The method of the invention described here above may further comprise an additional image analysis step and/or a visual analysis step for quantitative and/or qualitative determination of asbestos fibers. This additional step aims to provide a quantitative and/or a qualitative analysis of the asbestos fibers detected in the sample. Such a quantitative and/or a qualitative analysis may consist in known techniques such as image analysis, e.g. analysis of the images using fluorescence microscopy or colorimetric analysis using a standard range.

By the term "quantitative analysis", in the sense of the present invention, it is understood, the assessment of the quantity or concentration of asbestos in a sample.

By the term "qualitative analysis", in the sense of the present invention, it is understood, the assessment of the presence/absence of asbestos in a sample, in particular the presence/absence of asbestos specific type such as amphibole, chrysotile, crocidolite, amosite, tremolite, actinolite, anthophyllite, or other types of asbestos fibres.

By the term "visual analysis", in the sense of the present invention, it is understood, the determination of the presence or the absence of asbestos by the means of a visual assessment by comparing the sample appearance with a reference such as a standard range and/or a positive/negative control.

According to another aspect, the present invention also provides an asbestos-binding peptide comprising the sequence T G A A E V A M M S K R [SEQ ID NO:1]. This novel asbestos-binding peptide has 12 amino acids.According to the present invention, the asbestos-binding peptide may advantageously comprise a C-terminal amidation such as an amidation at the C-terminal end of the amino acid sequence.

The present invention is also about an asbestos-detection agent comprising at least one asbestos-binding peptide according to the invention, coupled to a reporter molecule or to a reporter amino acid sequence, possibly in tandem repeats, possibly by way of a linker selected from Poly-Gly or Gly-rich linkers the length of which may vary, including GGG, GGGS, and GGSSG; and wherein said reporter sequence is preferably selected from fluorescent protein, luciferase, alkaline phosphatase, beta galactosidase, beta-lactamase, diaphorase and peroxidase, maltose-binding protein, laccase, lipase, and glucoside hydrolase, and the reporter molecule is selected from fluorescent molecule or fluorescent dyes or pigments, such as fluorescein, Cy3, DyLight488, Alexa488, ATTO488, CF488A, DyLight550, CF555, Cy2, rhodamine green, rhodamine, Cy5, Alexa546, Alexa555, Alexa568, Alexa594, CF568, CF594 and DY547.

According to yet another aspect, the present invention relates to a novel asbestos-binding peptide that may be used as asbestos-detecting agent in accordance with the invention. Said at least one asbestos-binding peptide according to the invention may be coupled to a reporter molecule or fused to a reporter sequence.

Further, according to the invention, the above-defined peptide may be linked in tandem repeats.

Reporter molecules may advantageously be selected from fluorescent dyes or pigments, such as fluorescein, Cy3, DyLight488, Alexa488, ATTO488, CF488A, DyLight550, CF555, Cy2, rhodamine green, rhodamine, Cy5, Alexa546, Alexa555, Alexa568, Alexa594, CF568, CF594 and DY547 .

Reporter sequences may advantageously be selected from fluorescent protein, luciferase, alkaline phosphatase, beta-galactosidase, beta-lactamase, diaphorase, peroxidase, maltose-binding protein, laccase, lipase, and glucoside hydrolase.

According to a preferred embodiment, the asbestos-binding peptide according to the invention is fused to a reporter sequence or coupled to a reporter molecule by way of a linker. The linker may be selected from Poly-Gly or Gly-rich linkers the length of which may vary, including GGG, GGGS, GGSSG.

According to yet another aspect, the present invention further provides an asbestos detection kit for the detection of asbestos in a sample, comprising at least one asbestos-binding peptide and/or at least one asbestos detection agent, possibly in solution.

The relevant detection kit may advantageously further comprise revealing agent(s) or solution(s) and/or washing agent(s) or solution(s) and/or conditioning agent(s) or solution(s) and/or positive control(s) and/or the required hardware, such as beakers and pipettes, preferably one-way pipettes, for the performance of the method for the detection of asbestos as described above, that is, more specifically the contacting of at least one asbestos-binding peptide with the sample, and possibly means to separate at least one bound asbestos-binding peptide from unbound peptides, for instance for removing unbound peptides, such as a washing solution, hence allowing for detection of the presence of peptides bound to asbestos fibers.

Preferably, according to the invention, the revealing agent is a reporter substrate for colorimetric techniques or the revealing agent is a fluorescent probe for fluorescent techniques.

By the terms "revealing agent" or "revealing solution", it is meant in the sense of the present invention, an agent or a solution to reveal the presence of said at least one asbestos-binding peptide using for example a colorimetric or fluorescent technique. The revealing agent may contain a reporter substrate which is able to react with the reporter (for example an enzyme), said reporter being coupled to said at least one asbestos-binding peptide. The reporter substrate can for example be nitrocefins or derivates thereof, or derivates from beta-lactams. The revealing agent or revealing solution may be an aqueous solution comprising a buffer and/or a stabilizing agent.

By the terms "conditioning agent" or "conditioning solution", it is meant in the sense of the present invention, an agent or a solution to saturate the non-specific binding site and to introduce a competition between said at least one asbestos-binding peptide and the conditioning agent, in order to reduce unspecific binding. In particular, the conditioning agent contains a blocking or saturating agent such as Bovine serum Albumine (BSA), or caseine, or milk protein or trident in a buffer solution. The conditioning agent or the conditioning solution may be an aqueous solution comprising a buffer.

By the terms "positive control", it is meant in the sense of the present invention, a mean to assess the ability of said at least one asbestos-binding peptide to efficiently bind to a specific and selective binding site. In particular, the positive control can be for example a solution, a powder or a solid tablet containing a specific and selective binding site for said at least one asbestos-binding peptide. Preferably, according to the invention, the positive control comprises microcrystalline cellulose. More preferably, the positive control is a solid tablet.

By the terms "washing agent" or "washing solution" (or "rinsing solution"), it is meant in the sense of the present invention, an agent or a solution, in particular an aqueous solution, comprising a buffer.

A buffer according to the invention, for example a buffer present in a revealing solution, in a conditioning solution or in a washing solution, may be MES, MOPS, HEPES, TRIS, PBS, TBS or other known buffers.

In one embodiment the hardware of the asbestos detection kit according to the invention comprises a triple column system, wherein a first column is the testing column to introduce the sample, a second column is the positive control column to introduce the positive control and, a third column is the negative control column.

Advantageously, a collection tank/reservoir is located beneath the triple column system to collect the different solutions. Advantageously, a mobile organ is placed between the collection tank/reservoir and the columns in order to control the solution flows from the column to the reservoir. Therefore, said mobile organ allows the kit user to empty the column content in order to perform the asbestos detection technique.

More advantageously, said mobile organ is handled by the kit user without access to the column content in order to limit/avoid asbestos manipulation. Preferably, the mobile organ is a pulling vane which, when activated, let the column content flows in the reservoir located beneath the columns.

In another embodiment, the asbestos detection kit may comprise additional means to detect one or more types of asbestos fibers as for example, amphibole, chrysotile, crocidolite, amosite, tremolite, actinolite, anthophyllite, or other types of asbestos fibers known by the person skilled in the art.

Preferably, said additional means to detect one or more types of asbestos fibers, implies an additional image analysis step and/or an additional visual analysis step such as fluorescent microscopy and/or colorimetric techniques.

Advantageously, the detection kit of the present invention is particularly easy to use, even by rather low qualified personnel, and is capable of providing test results in a short period of time. It has been established so far that results may be obtained within less than approx. 1 hour, preferably less than approx. 0.5 hour, more preferably less than approx. 0.25 hour.

The present invention is also about the use of a kit according to the invention to detect asbestos in a sample.

## Brief Description of the Figures

The invention will be described in more details herein below with reference to the attached figure.

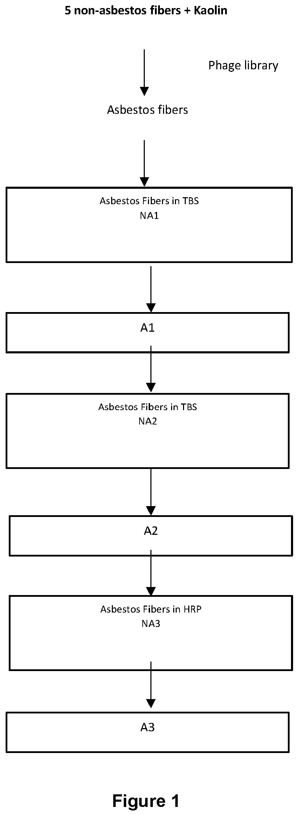

Fig. 1 describes an example of the phage procedure applied to obtain SEQ ID NO:1.

## Example

Having generally described the invention, the same will be more readily understood by reference to the following example, which is provided by way of illustration and is not intended as being limiting.

1) Procedure to obtain SEQ n°1, also referred to as ASBEST6

To obtain SEQ ID NO:1, different cycles of panning of phage display were performed, as shown schematically on Figure 1.

In this example, as a summary, the phage procedure proceeds according to the following steps. The phage library is applied to the counter selection sample composed of 5 non-asbestos fibers and kaolin. Non-asbestos-binding peptides forming the supernatant constitute the new library. Then, the new library produced during the counter selection step, is applied to the selection sample composed of various asbestos fibers in TBS (Tris-buffered Saline). Asbestos-binding peptide are extracted and consist of NA1 (non-amplificated first library). Consequently, A1 (amplificated first library) is obtained after amplification and purification of NA1. Then, A1 is applied to the selection sample in TBS and NA2 (non-amplificated second library) is extracted. Consequently, A2 is obtained after amplification and purification of NA2. Finally, A2 is applied to the selection sample in HRP-conjugate Stabilizer (D-tek) and NA3 (non-amplificated third library) is extracted. Consequently, A3 is obtained after amplification and purification of NA3.

## Round 1: Production of the first library (A1)

A first round allowed to perform a counter-selection against 6 non-asbestos fibers/materials and the plastic Eppendorf used for the procedure (the fibers used were: Kaolin, Rockwool, sized fiberglass, fiberglass, cellulose and Kevlar), and a selection against various asbestos fibers (Chrysotile, Crocidolite, Amosite, Tremolite, Actinolite, Anthophyllite and a treated asbestos, still asbestiform). The procedure was as follows:

*Counter-selection step:* A plastic tube containing a composition of 180 mg of a homogeneous mix of the non-asbestos fibers/materials, especially, Kaolin, Rockwool, sized fiberglass, fiberglass, cellulose and Kevlar (30 mg of each), in 5 ml Buffer composed of 50 mM Tris-HCl (pH 7.5) and 150 mM NaCl, was incubated for one hour under soft rotation at room temperature. Then, 50 µl of a commercial library Ph.D.-12 ($1 \times 10^{13}$ phages) (Ph.D.™-12 Phage Display Peptide Library, NEB, a combinatorial library of random dodecapeptides fused to a minor coat protein (pIII) of M13 phage) were added onto the composition contained in the plastic tube (Eppendorf). The plastic tube (Eppendorf) was then rotated by an agitator for one hour at room temperature in order to obtain sufficient time for phage-fibers/materials interaction. Then, after one minute of centrifugation at 14.000 rpm at 22°C of the plastic tube, 5 ml of supernatant were collected to be used as initial library for selection described in the following step. The collected supernatant corresponding to the initial library, contains the non-attached phage exposing peptide, meaning the peptide exposed to the sample containing non-asbestos fibers/materials such as Kaolin, Rockwool, sized fiberglass, fiberglass, cellulose and Kevlar, does not interact with those materials and plastic.

*Selection step:* In parallel, the mineral solution was produced in a tube containing 150 mg of a homogeneous mix of asbestos fibers (25 mg of each) in 5 ml TBS buffer (50 mM Tris-HCl (pH 7.5), 150 mM NaCl). After one hour incubation of the tube under soft rotation at room temperature, and one minute of centrifugation at 14.000 rpm at 22°C, the buffer solution was poured-off from the Eppendorf tube. Then 5ml of blocking buffer (50 mM Tris-HCl (pH 7.5), 150 mM NaCl and 5mg/ml of BSA) was added in the tube and it was incubated for one hour at 4 °C. After centrifugation of the tube, the blocking solution was discarded, and the remaining sample contained in the tube was washed rapidly 6 times with TBS. Then, the 5 ml of the initial library obtained from the counter-selection procedure was used to resuspend the fibers. The mineral-phage solution containing asbestos material and the initial library was rotated by an agitator for one hour in order to obtain sufficient time for phage-mineral interaction.

*Washing step*: After the above described selection step, the supernatant was discarded (non-attached peptide-phage on materials) by performing ten washing cycles, with 5 ml TBS containing 0.1 % tween 20, in order to remove the non-specific phage binding on the asbestos fibers. Phages that strongly bond to the target substrate were retained, while the non-binding phages were washed away. For each washing cycle, the supernatant was discarded.

*Elution step*: Strongly bound phages were recovered from the mineral surface through elution. The strong interaction between phage and material was disrupted using a low pH buffer solution, as 0.2 M Glycine-HCl (pH 2.2). 5 ml of low pH elution buffer solution was added into the microcentrifuge tube. The microcentrifuge tube was submitted to agitation for 5 minutes to elute the bound phages from mineral surface. The low pH buffer solution was neutralized with a solution composed of 750 µl of 1 M Tris-HCl, pH 9.1. The collected supernatant was named **NA1** for non-amplificated first library.

*Amplification and purification steps of NA1*: NA1 sample obtained from the elution step (eluted phages) were infected into the host strain *E. coli* ER2738 and amplified. Before the beginning of amplification period, an overnight culture of ER2738 was grown. *E. coli* host strain ER2738 used in amplification is a robust F+ strain with a rapid growth rate and is particularly well-suited for M13 propagation. ER2738 is a recA+ strain and the F factor of ER2738 contains a mini-transposon, which confers tetracycline resistance. A solution containing 20 ml of LB medium (lysogeny broth) in a 250 ml Erlenmeyer flask was inoculated with 200 µL overnight E. coli culture. Then, 200 µL of NA1 was added to the solution contained in the Erlenmeyer. Then, the solution contained in the Erlenmeyer was submitted to shaking at 37°C, 250 rpm during four to five hours. After the above mentioned amplification step, the amplified phages were purified from host cell according to the following procedure. First, E.coli-phage culture was transferred to 50 ml sterilized centrifuge tubes to be submitted to centrifugation at 8000 rpm for 10 min to remove the bacterial cells. The supernatant was transferred to a fresh 50 ml sterilized centrifuge tube and the centrifugation was repeated twice. The resultant 16 ml of supernatant were transferred to a new tube and 4 mL of 2.5 M NaCl/20 % PEG-8000 (w/v) was added. Briefly, the solution was mixed and the phages precipitated overnight at 4°C. The phages were pelleted by centrifugation at 12000 g for 15 min. The supernatant was discarded and the pellet resuspended in 1 mL TBS. A new centrifugation at 8000 rpm for 10 min was realized to remove any cell debris. The supernatant was transferred to a fresh tube and 200 µL of 2.5 M NaCl/20% PEG-8000 were added. Incubation was performed on ice for 60 min. The tube was submitted to centrifugation at 12000 -14000 rpm in a benchtop centrifuge for 10 min. the supernatant was discarded, the tube was submitted briefly to centrifugation again to remove the remaining supernatant with pipette. The pellet was resuspended in 200 µL TBS, this is **A1** for amplified first library.

*Titration of A1:* This experiment was performed to estimate the phage titers at the end of each biopanning round. The blue-white screening experiment has three fundamental steps, namely, preparation of Xgal/IPTG plates, serial dilution of eluted phage samples, and estimation of phage titers. Preparation of LB-Xgal/IPTG plates consists of adding187 µl Xgal/IPTG in 150 ml liquid warm LB agar to be contained in 90 mm plastic sterile petri dish. Plates were wrapped with parafilm and stored at 4°C in the dark for a maximum of one month. Then the serial dilution of phages contained in A1 was performed. 90 µl TBS buffer (without detergent) and 10 µl phages A1 was diluted until 24 times. Finally, we proceeded to the calculation of Phage Titers. After serial dilutions, LB-Xgal/IPTG plates that were prepared previously and stored at 4°C, were put at room temperature. 3 ml of top agar were melted and aliquoted into 15 ml cell culture tube and these tubes were put in 55°C water bath to prevent the solidification until other processes were carried out. E. coli ER2738 (1:250) from overnight culture were inoculated into 5 ml LB medium in falcon tube. The culture was incubated until mid-log phase (OD600 ~ 0.5) at 37°C and 250 rpm. After incubation period, 200 $\mu$l E. coli ER2738 culture and 10 $\mu$l of the serial dilution sample of phages A1 were put into 1.5 ml eppendorf tube. The mixture was added into 3 ml melt top agar and poured onto LB-Xgal/IPTG petri dishes. All petri dishes were kept up-side down and incubated at 37°C for overnight. After incubation period, blue plaques were obtained. The plate with 30 to100 plaques from each diluted solution were chosen to calculate the amount of phage with the equation as follow:

$$\text{The amount of phage (pfu)} = \frac{\text{The number of plaques}}{\text{The volume of diluted phage solution (ml)}} \times \text{Dilution factor}$$

pfu: plaque forming unit

According to phage titers at each round, phage amount was determined to generate a phage pool at a concentration of $1 \times 10^{13}$ phages for the next biopanning round.

## Round 2 : Production of the second library (A2)

A second round was performed and consists of a second cycle of selection against the same asbestos fibers according to a similar procedure as round 1 except the absence of counter-selection.

Selection step was performed as in round 1 starting with the selection step, except that after washing 6 times with TBS, 50 $\mu$l of A1 was added to the mineral solution containing 5 ml of TBS and asbestos materials. The mineral-phage solution was rotated by an agitator for one hour in order to obtain sufficient time for phage-mineral interaction. Washing and elution steps were identical to round 1 except the use of TBS containing 0.5 % tween 20. The supernatant recovered was named **NA2** for non-amplificated second library.

Amplification and purification steps of NA2 was performed as for round 1 and referred to as **A2** for amplified second library. Titration of A2 was performed as for round 1 and phage amount was determined as explained above to generate a phage pool at a concentration of $1 \times 10^{13}$ phages for the next biopanning round.

## Round 3 : Production of the third library (A3)

A third round was performed and consists of a third cycle of selection against the same asbestos fibers according to a similar procedure as round 1 except the absence of counter-selection.

Selection step was performed as in round 1 except that the buffer was HRP-conjugate Stabilizer (D-Tek) instead of TBS and after washing 6 times with HRP, 50 $\mu$l of A2 was added to the mineral solution containing 5 ml of HRP and asbestos materials. The mineral-phage solution was rotated by an agitator for one hour in order to obtain sufficient time for phage-mineral interaction. Washing and elution steps were identical to round 1 except the use of HRP containing 0.8% tween 20. The supernatant recovered was named **NA3** for non-amplificated third library.

Amplification and purification steps of NA3 was performed as for round 1 and referred to as **A3 for amplified third library.** Titration of A3 was performed as for round 1 and phage amount was determined as explained above to generate a phage pool at a concentration of $1 \times 10^{13}$ phages.

## 2) Sequencing to determine the selected peptide sequence on A1, A2 and A3

*Purification of sequencing templates:* An overnight culture of ER2738 was diluted 1:100 in LB medium. 1 ml of diluted culture, one for each clone to be characterized, was dispensed into culture tubes. With a sterile wooden stick, it was stabbed a blue plaque from a tittering plate (of A1, A2 and A3) and transferred to a tube containing the diluted culture. The tubes were incubated at 37°C with shaking for 4.5-5 hours. The cultures were transferred to microcentrifuge tubes and centrifuged at 14,000 rpm for 30 seconds. 500 $\mu$L of the phage-containing supernatant were transferred to a fresh microcentrifuge tube. 200$\mu$L of 20% PEG/2.5 M NaCl were added for 10 min and mixed at room temperature. Then, after centrifuged at 14,000 rpm for 10 minutes at 4°C, the supernatant was discarded. The pellet was suspended thoroughly in 100 $\mu$L of iodide buffer, then 250 $\mu$L of ethanol was added and incubated 10 minutes at room temperature. It was spun in a microcentrifuge tube at 14,000 rpm for 10 minutes at 4°C and the supernatant was discarded. The pellet was washed with 0.5 ml of 70% ethanol, re-spun, and the supernatant was discarded again. The pellet was briefly dried under vacuum. The pellet was suspended in 30 $\mu$l of sterile H20. The product was quantitated by nanodrop.

*DNA sequencing:* The platform Genomic facility of the University of Liege provided service DNA sequencing for single strain DNA of M13 phage clones. Primers purchased from New England Biolabs Ph.D.-12 Phage Display Peptide Library Kit was used in PCR. DNA samples were sequenced by using AB Solid System.

EP 4 700 389 A1

*Determination of sequence of the peptides:* The chromatograms obtained by sequencing were analyzed by Chromas Lite. For the DNA of M13 Phage in Ph.D.-12 phage library, the DNA codes are the same except 12-mer peptide-gIII fusion for each phage. In order to find out the 12- mer peptide gill fusion, two segments of DNA sequences in the immediate vicinity of gill fusion domain were used and aligned with any DNA sequence of phage clones by using Nucleotide alignment in Basic Local Alignment Search Tool (BLAST) from National Center for Biotechnology Information (NCBI): http://blast.ncbi.nlm.nih.gov/. After obtaining the DNA sequence of gill fusion, DNA codes of gill fusion can be translated into amino acid sequences of 12-mer peptides. Thus ASBEST6 sequence was identified as TGAAEVAMMSKR, it represented 9 peptides out of 30 for library A1, 22 peptides out of 30 for library A2 and A3.

**3) Validation of the recognition and selectivity of peptide ASBEST6:**

The following steps were performed for the detection of asbestos using fluorescence microscopy:

Step 1 is the sample saturation. A composition containing 15 mg of each material (Kaolin, Rockwool, sized fiberglass, fiberglass, cellulose, Kevlar, Chrysotile, Crocidolite, Amosite, Tremolite, Actinolite Anthophyllite and treated asbestos still asbestiform) was saturated with 1 mL of Mops 50 mM pH8, KNO3 150 mM, BSA 1%. Then, according to procedure A, the composition was processed through orbital shaking, then it was centrifuged 5 min at 10,000 rpm, and finally, the supernatant was removed.

Step 2 is a first rinsing. The material obtained according to step 1, was rinsed with 1 mL of Mops 50 mM pH8, KNO3 150 mM, then it was vortexed and then, the procedure A was applied.

Step 3 is the peptide binding. 1 mL of 0.1 mg/mL of the fluorescent peptide (ASBEST6 with sequence TGAAEVAMMSKR-Lys(FITC) or prior art peptide) in 50 mM Mops pH8, 150 mM KNO3 was incubated with the material obtained according to step 2, then the mix was vortexed and finally, the procedure A was applied.

Step 4 is a second rinsing. The material obtained according to step 3 was rinsed with 1 mL of Mops 50 mM pH8, KNO3 150 mM and 2% of Tween 20, then the mix was vortexed and finally, the procedure A was applied.

Step 5 is a third rinsing. The material obtained according to step 4 was rinsed with 1 mL of Mops 50 mM pH8, KNO3 150 mM, then the mix was vortexed and finally, the procedure A was applied.

Finally, the material obtained according to step 5 was observed using visible and fluorescent microscopy.
In these conditions, it was shown that the fluorescent ASBEST6 referring to [SEQ ID NO:1] recognizes Chrysotile, Crocidolite, Amosite, Tremolite, Actinolite Anthophyllite (green fluorescence) while it does not recognise Kaolin, Rockwool, sized fiberglass, fiberglass, cellulose and Kevlar (no fluorescence visible). An excellent selectivity was obtained.

**4) Comparison with the prior art sequence, CHRYSO 8 comprising the sequence G H C S H P T, as disclosed in EP-3770599.**

**4.1 Recognition of non-asbestos samples with fluorescent peptides**

Selectivity of SEQ ID NO:1 has been tested with fluorescence against non-asbestos samples, in comparison to the prior art peptide referred as CHRYSO 8 comprising the sequence G H C S H P T. Results from this comparison are displayed in table 1.

Table 1: Comparison of selectivity against non-asbestos samples between the prior art peptide, CHRYSO 8, and SEQ ID NO:1 peptide tested with fluorescence.

| Name/description | Matrix | Prior art peptide CHRYSO8 (GHCSHPT) | SEQ ID NO:1 |
|---|---|---|---|
| Kaolin | Pure fibers | +++ | none |
| Rockwool | Pure fibers | + | none |
| Sized glass fibers | Pure fibers | ++ | none |
| Virgin glass fibers | Pure fibers | + | none |
| Cellulose | Pure fibers | +++ | none |

(continued)

| Name/description | Matrix | Prior art peptide CHRYSO8 (GHCSHPT) | SEQ ID NO:1 |
|---|---|---|---|
| Kevlar | Pure fibers | + | none |
| Plaster non asbestos | Home-made | +++ | + |
| FO - plasters insulation NA | Real material | +++ | + |
| Ceiling (artif. miner. fibers + perlite) NA | Real material | +++ | + |
| Fiber cement (cellulose) - fregus | Real material | +++ | + |
| Under fiber cement roof | Real material | ++ | none |
| Legend : none = not selective, + = low selectivity, ++ = good selectivity, +++ = very good selectivity. | | | |

From Table 1, it appears that SEQ ID NO:1 presents a better selectivity than peptide of the prior art since it does not recognise or only very weakly recognize materials without asbestos.

**4.2 Recognition of asbestos samples with fluorescent peptides**

Selectivity of SEQ ID NO:1 has been tested with fluorescence against asbestos samples, in comparison to the prior art peptide referred as CHRYSO 8 comprising the sequence G H C S H P T. Results from this comparison are displayed in table 2.

Table 2: Comparison of selectivity against asbestos samples between the prior art peptide, CHRYSO 8, and SEQ ID NO:1 peptide tested with fluorescence.

| Name/description | Matrix | Prior art peptide CHRYSO8 (GHCSHPT) | SEQ ID NO:1 |
|---|---|---|---|
| Chrysotyle | Pure fibers | +++ | ++ |
| Crocidolite | Pure fibers | + | none |
| Amosite | Pure fibers | ++ | none |
| Tremolite | Pure fibers | + | ++ |
| Actinolite | Pure fibers | +++ | + |
| Resin with 0.1% of chrysotile | Home-made | none | ++ |
| AKT-012 cement and organic fibers with very few chrysotile | Real material | + | ++ |
| AKT22 roofing | Real material | + | ++ |
| Legend : none = not selective, + = low selectivity, ++ = good selectivity, +++ = very good selectivity. | | | |

From Table 2, it appears that SEQ ID NO:1 is less sensitive than the prior art peptide to recognize pure asbestos fibers, however when present in a material, asbestos is better detected with SEQ ID NO:1.

**4.3 Recognition of non-asbestos samples as tested by colorimetry on chimera peptides**

Selectivity of SEQ ID NO:1 has been tested by colorimetry against non-asbestos samples, in comparison to the prior art peptide referred as CHRYSO 8 comprising the sequence G H C S H P T. Results from this comparison are displayed in table 3.

Table 3: Comparison of selectivity against non-asbestos samples between the prior art peptide, CHRYSO 8, and SEQ ID NO:1 peptide tested by colorimetry.

| Name/description | Matrix | Prior art peptide CHRYSO8 (GHCSHPT) | SEQ ID NO:1 |
|---|---|---|---|
| Kaolin | Pure fibers | Purple | Yellow |
| Microtalc | Pure fibers | Purple | Yellow |

(continued)

| Name/description | Matrix | Prior art peptide CHRYSO8 (GHCSHPT) | SEQ ID NO:1 |
|---|---|---|---|
| Kevlar fibers - centexbel | Pure fibers | Purple | Yellow |
| Fiber Cerawool blanket | Pure fibers | Purple | Yellow |
| Kevlar fibers | Pure fibers | Purple | Yellow |
| Sommière earth | ground | Purple | Gray |
| Green clay | ground | Gray | Yellow |
| Montmorillonite | ground | Purple | Yellow |
| Fiber cement (cellulose) tecos | Real material | Gray | Yellow |
| Fiber cement (cellulose) fregus | Real material | Gray | Yellow |
| Ceiling with organic fibers | Real material | Gray | Yellow |
| Plasters insulation | Real material | Gray | Yellow |
| Ceiling with mineral fibers and perlite | Real material | Gray | Yellow |
| Asbestos-free vinyl | Real material | Gray | Yellow |
| Legend : yellow = no recognition / selectivity ; gray = low recognition / selectivity ; purple = good recognition / selectivity. | | | |

From Table 3, it appears that SEQ ID NO:1 presents a better selectivity than the peptide of the prior art, CHRYSO 8 comprising the sequence GHCSHPT, since it does not recognize (yellow color) or only very slightly (gray color) recognizes materials without asbestos, while the peptide of the prior art recognizes (purple color) materials without asbestos. Hence, it has less false positive samples.

### 4.4 Recognition of asbestos samples as tested by colorimetry on chimera peptides

Selectivity of SEQ ID NO:1 has been tested by colorimetry against asbestos samples, in comparison to the prior art peptide referred as CHRYSO 8 comprising the sequence G H C S H P T. Results from this comparison are displayed in table 3.

Table 3: Comparison of selectivity against asbestos samples between the prior art peptide, CHRYSO 8, and SEQ ID NO:1 peptide tested by colorimetry.

| Name/description | Matrix | Prior art peptide CHRYSO8 (GHCSHPT) | SEQ ID NO:1 |
|---|---|---|---|
| Chrysotile | Pure fibers | Gray | Purple |
| Cement with 0,1% Chrysotile | Home-made | Yellow | Purple |
| Cement- Amosite 5% | Home-made | Yellow | Purple |
| Cement with 5% Chrysotile | Home-made | Yellow | Purple |
| Resin with 15% Chrysotile | Home-made | Yellow | Purple |
| Resin with 5% Chrysotile | Home-made | Yellow | Purple |
| Resin with 1% Chrysotile | Home-made | Yellow | Purple |
| Resin with 0,1% Chrysotile | Home-made | Yellow | Purple |
| Fibro Cement-Chrysotile bonded material-board 15% | Real material | Yellow | Purple |
| ATA 21-012 (Cementorganic fibers and small amount of chrysotile) | Real material | Gray | Purple |
| AKT05 Black glue + screed | Real material | Gray | Purple |
| AKT22 Roofing Hannut Plant | Real material | Yellow | Purple |
| FloorFlex Vynil | Real material | Yellow | Purple |

(continued)

| Name/description | Matrix | Prior art peptide CHRYSO8 (GHCSHPT) | SEQ ID NO:1 |
|---|---|---|---|
| Asbestos Rope - Boiler Gasket | Real material | Gray | Purple |

| Legend : yellow = no recognition ; gray = low recognition ; purple = good recognition. |
|---|

From Table 4, it appears that SEQ ID NO:1 gives better results than the peptide of the prior art, CHRYSO 8, comprising the sequence GHCSHPT, since it always recognizes (purple color) materials with asbestos, on pure fibers, on home-made materials and on real materials. Hence, it has less false negative samples.

**Claims**

1. A method for the detection of asbestos in a sample, comprising:

    (a) contacting a sample with at least one asbestos-binding peptide comprising the sequence T G A A E V A M M S K R [SEQ ID NO:1]; and
    (b) detecting the presence of said at least one asbestos-binding peptide bound to asbestos fibers.

2. The method according to claim 1, further comprising a step to separate said at least one bound asbestos-binding peptide from unbound asbestos-binding peptides, such as a washing step.

3. The method according to claims 1 or 2, wherein detecting the presence of said at least one asbestos-binding peptide bound to asbestos fibers comprises fluorescence techniques.

4. The method according to claims 1 or 2, wherein detecting the presence of said at least one asbestos-binding peptide bound to asbestos fibers comprises colorimetric techniques.

5. The method according to any preceding claims 1 to 4, wherein one or more asbestos-binding peptide is coupled, possibly in tandem repeats, to a reporter sequence (amino acid sequence), such as a reporter sequence selected from fluorescent protein, luciferase, alkaline phosphatase, beta galactosidase, beta-lactamase, diaphorase and peroxidase, maltose-binding protein, laccase, lipase, and glucoside hydrolase, or coupled, possibly in tandem repeats, to a reporter molecule selected from fluorescent dyes or pigments, such as fluorescein, Cy3, DyLight488, Alexa488, ATTO488, CF488A, DyLight550, CF555, Cy2, rhodamine green, rhodamine, Cy5, Alexa546, Alexa555, Alexa568, Alexa594, CF568, CF594 and DY547.

6. The method according to any preceding claims 1 to 5, wherein at least one asbestos-binding peptide is coupled to an enzyme such as a reporter sequence enzyme, the method comprising an additional step wherein one or more reporter substrate contacts said at least one asbestos-binding peptide coupled to an enzyme.

7. The method according to any preceding claims 1 to 6, wherein the sample is a sample of construction material, an air sample, a ground sample or a sample of biological material.

8. The method according to any preceding claims 1 to 7, further comprising an additional image analysis step and/or a visual analysis step for quantitative and/or qualitative determination of asbestos fibers.

9. An asbestos-binding peptide comprising the sequence T G A A E V A M M S K R [SEQ ID NO:1].

10. The asbestos-binding peptide according to claim 9 comprising a C-terminal amidation.

11. An asbestos-detection agent comprising at least one asbestos-binding peptide according to claims 9 or 10, coupled to a reporter molecule or to a reporter amino acid sequence, possibly in tandem repeats, possibly by way of a linker selected from Poly-Gly or Gly-rich linkers the length of which may vary, including GGG, GGGS, and GGSSG; and wherein said reporter sequence is preferably selected from fluorescent protein, luciferase, alkaline phosphatase, beta

galactosidase, beta-lactamase, diaphorase and peroxidase, maltose-binding protein, laccase, lipase, and glucoside hydrolase, and the reporter molecule is selected from fluorescent molecule or fluorescent dyes or pigments, such as fluorescein, Cy3, DyLight488, Alexa488, ATTO488, CF488A, DyLight550, CF555, Cy2, rhodamine green, rhodamine, Cy5, Alexa546, Alexa555, Alexa568, Alexa594, CF568, CF594 and DY547.

12. A kit for the detection of asbestos in a sample, comprising a least one asbestos-binding peptide according to claims 9 or 10 or at least one asbestos detection agent according to claim 11, possibly in solution.

13. A kit for the detection of asbestos in a sample according to claim 12, further comprising a conditioning agent, and/or a washing solution, and/or a revealing agent and/or a positive control.

14. A kit for the detection of asbestos in a sample according to claim 13, wherein the revealing agent is a reporter substrate for colorimetric techniques or the revealing agent is a fluorescent probe for fluorescent techniques.

15. Use of a kit according to any preceding claims 12 to 14 to detect asbestos in a sample.

**5 non-asbestos fibers + Kaolin**

↓                    Phage library

Asbestos fibers

↓

```
┌─────────────────────────────┐
│   Asbestos Fibers in TBS    │
│            NA1              │
│                             │
└─────────────────────────────┘
```

↓

```
┌─────────────────────────────┐
│             A1              │
└─────────────────────────────┘
```

↓

```
┌─────────────────────────────┐
│   Asbestos Fibers in TBS    │
│            NA2              │
│                             │
└─────────────────────────────┘
```

↓

```
┌─────────────────────────────┐
│             A2              │
└─────────────────────────────┘
```

↓

```
┌─────────────────────────────┐
│   Asbestos Fibers in HRP    │
│            NA3              │
│                             │
└─────────────────────────────┘
```

↓

```
┌─────────────────────────────┐
│             A3              │
└─────────────────────────────┘
```

**Figure 1**

# EUROPEAN SEARCH REPORT

**Application Number**

EP 24 19 5646

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 117 069 802 A (UNIV ZHENGZHOU) 17 November 2023 (2023-11-17) | 9,12 | INV. G01N33/53 |
| A | * P-2 in Fig. 3 * | 1-8,10, 11,13-15 | C07K7/06 C07K7/08 |
| A | EP 1 953 549 A1 (NAT UNIVERSITY OF CORP HIROSHI [JP]) 6 August 2008 (2008-08-06) * the whole document * | 1-15 | |
| A | WO 2017/029190 A1 (SYMBIOSE BIOMATERIALS SA [BE]) 23 February 2017 (2017-02-23) * the whole document * | 1-15 | |
| A | TAKENORI ISHIDA ET AL: "Molecular Engineering of a Fluorescent Bioprobe for Sensitive and Selective Detection of Amphibole Asbestos", PLOS ONE, vol. 8, no. 9, 27 September 2013 (2013-09-27), page e76231, XP055316223, DOI: 10.1371/journal.pone.0076231 * the whole document * | 1-15 | |
| A | TAKENORI ISHIDA ET AL: "Selective Detection of Airborne Asbestos Fibers Using Protein-Based Fluorescent Probes", ENVIRONMENTAL SCIENCE & TECHNOLOGY, vol. 44, no. 2, 15 January 2010 (2010-01-15), pages 755-759, XP055550680, US ISSN: 0013-936X, DOI: 10.1021/es902395h * the whole document * | 1-15 | |

**TECHNICAL FIELDS SEARCHED (IPC)**

G01N
C07K

The present search report has been drawn up for all claims

| Place of search | Date of completion of the search | Examiner |
|---|---|---|
| Munich | 4 February 2025 | Jacques, Patrice |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 24 19 5646

This annex lists the patent family members relating to the patent documents cited in the above-mentioned European search report.
The members are as contained in the European Patent Office EDP file on
The European Patent Office is in no way liable for these particulars which are merely given for the purpose of information.

04-02-2025

| Patent document cited in search report | | Publication date | Patent family member(s) | | Publication date |
|---|---|---|---|---|---|
| CN 117069802 | A | 17-11-2023 | NONE | | |
| EP 1953549 | A1 | 06-08-2008 | EP | 1953549 A1 | 06-08-2008 |
| | | | JP | 5011544 B2 | 29-08-2012 |
| | | | JP | 5464385 B2 | 09-04-2014 |
| | | | JP | 2012108163 A | 07-06-2012 |
| | | | JP | WO2007055243 A1 | 30-04-2009 |
| | | | US | 2009098578 A1 | 16-04-2009 |
| | | | WO | 2007055243 A1 | 18-05-2007 |
| WO 2017029190 | A1 | 23-02-2017 | EP | 3335044 A1 | 20-06-2018 |
| | | | EP | 3770599 A1 | 27-01-2021 |
| | | | WO | 2017029190 A1 | 23-02-2017 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 1953549 A

- EP 3770599 A

### Non-patent literature cited in the description

- **ISHIDA et al.** Selective detection of airborne asbestos fibers using protein-based fluorescent probes. *Environ Sci Technol.*, 15 January 2010, vol. 44 (2)

- **T ISHIDA et al.** Molecular Engineering of a Fluorescent Bioprobe for Sensitive and Selective Detection of Amphibole Asbestos. *Plos One*, September 2013, vol. 8 (9), e76231
- **C TAMERLER et al.** Molecular biomimetics: utilizing nature's molecular ways in practical engineering. *Acta biomaterialia*, 2007, vol. 3 (3), 289-99